Europäisches Patentamt

European Patent Office

Office européen des brevets

(19)

(11) Publication number: **0 009 543**
**B1**

(12) **EUROPEAN PATENT SPECIFICATION**

(45) Date of publication of patent specification: **08.12.82**

(51) Int. Cl.³: **A 61 M 16/00**

(21) Application number: **79102287.4**

(22) Date of filing: **05.07.79**

(54) Nested hollow fiber humidifier.

(30) Priority: **12.07.78 US 923905**
**08.06.79 US 46943**

(43) Date of publication of application:
**16.04.80 Bulletin 80/8**

(45) Publication of the grant of the patent:
**08.12.82 Bulletin 82/49**

(84) Designated Contracting States:
**AT CH DE FR GB IT NL SE**

(56) References cited:
**DE - A - 2 617 985**
**DE - A - 2 703 892**
**US - A - 4 010 748**
**US - A - 4 086 305**
**US - A - 4 098 852**

(73) Proprietor: **Jackson, Richard R., Dr.**
**8, Trinity Road**
**Marblehead, Massachusetts 02138 (US)**

(72) Inventor: **Jackson, Richard R., Dr.**
**8, Trinity Road**
**Marblehead, Massachusetts 02138 (US)**

(74) Representative: **Moser, Herbert, Dr.-Ing.**
**Nowackanlage 15**
**D-7500 Karlsruhe (DE)**

Courier Press, Leamington Spa, England.

Nested hollow fiber humidifier

Background of the invention

This invention relates to a humidifier for humidifying and delivering gases such as air or oxygen directly to a patient or to a person in good health. It constitutes an improvement over my prior patents U.S. 3,616,796; 3,871,373; 3,912,795 and to later efforts of Dobritz, U.S. 4,010,748 and 4,086,305.

According to my prior patents, a new type of medical humidifier is provided which operates according to the diffusion principle in which water vapor from a water supply permeates a wall or membrane and enters a stream of breathing air while the water supply is maintained separate from the air stream by the wall. Such a diffusion humidifier offers a number of potential advantages over other methods of humidification, but no satisfactory form for its manufacture has been found. To be suitable for production and wide use I have recognized that numerous difficult criteria should be met. As the humidifier is to be connected directly to the airway of a patient and life-supporting air is to be channeled through it, it should have low airflow resistance, preferably so low that the patient can breath through the humidifier without assistance (e.g. with a flow resistance comparable to a standard endotracheal tube). It should be capable of fail-safe operation to avoid any substantial risk that the patient might inhale large volumes of gross (i.e. liquid) water. It should be of small size to avoid introducing a large compressible air volume in the path and to enable placement close to the mouth, for example as a tubular component of a breathing circuit. It should have a long shelf life. And it should be of simple and rugged construction and of such low cost as to be disposable.

In US Patent 4,098,852 the use of hollow fibres is described to promote heat exchange between a liquid and a gas. Such arrangement should be used in evaporation-type refrigerators, air-conditioners and presumably in room humidifiers. This older specification teaches positive pressure across the walls of the hollow fibres such that the absolute pressure of the liquid is substantially greater than the absolute pressure of the gas. It would be not advisable to transfer such arrangement to a respiratory flow path because its structure fails to meet many of the essential criteria and could not exclude the entering of a dangerous amount of water in the lungs if an accidental rupture of fibres occurs.

DE—A—26 17 985 discloses a humidifier for a respiratory flow path but only with small bore fibres without incorporating of a wetting agent and does not give any teaching to expose the fibres to water under relatively negative pressure.

At least, DE—A—27 03 892, published after the claimed priority date, shows a humidifier for a respiratory flow path having an air-transmitting housing with a bundle of U-like hollow fibres through which the water streams. Such arrangement also could not fulfill required conditions for efficiency and safety.

Summary of the invention

According to the invention the humidifier for a respiratory flow path, especially a medical humidifier, comprising a water chamber including a bundle of discrete elongated air-transmitting hollow fibres forming a compact nest and connected in parallel by inlet and outlet connections and having walls of a substance permeable to water vapor, whereby the water-filled chamber surrounds the effective length of the fibres is improved by the following features:

a) The fibres incorporate a wetting agent

b) The fibres having a bore greater than 1,27 mm (0.05 inch)

c) and means being provided to expose the exterior of the fibres to water under relatively negative pressure.

The fibres are arranged to divide dry inhalation air (here the word "air" is intended to include pure or diluted oxygen) for a person or a patient into a series of air-flow filaments for humidification by water vapor that permeates walls of the fibres from the surrounding water under negative pressure.

By this means a humidifier is provided which can achieve full humidification of air in a practical, low-flow-resistance compact unit that can be disposable.

According to important further aspects of the invention, said hollow fibres have a wall thickness that is 10% or less of the bore of said fibres.

A further improvement may be achieved by an embodiment wherein the substance of the fibres has a transmission characteristic from 4,8 kg water per $m^2$ per hour of fibres wall area per 0,127 mm wall thickness, with water at 37.5°C, at 103 Torr negative pressure on the exterior of said fibres and with a flow of anhydrous oxygen at an aggregate rate of 200 litres per minute flowing in parallel through a bundle of fibres of 127 mm length.

The fibres have advantageously an internal diameter of less than 1,78 mm (.070 inch). The substance of the fibres is selected from the group consisting of polysulfones and acrylic copolymers. In a further aspect of the invention the wetting agent is glycerine or dioctyl sodium sulfo succinate.

In a preferred embodiment an air flow resistance value R lies in the range of 52 and 134. For fibres of constant diameter

$$R = \frac{L}{D^4} \times \frac{1}{N}$$

where L is the air-transmitting length and D the internal diameter of the fibres in cm, and N is the number of the fibres.

Such humidifier could be improved by a number of fibres in the range of 100 to 200.

In an advantageous construction the humidifier has an effective length of the fibres of 12,7 cm.

For such humidifier it may be advantageous that the walls of said fibres are impermeable to liquid water under operating conditions and that said walls are 0.127 mm (0.005 inch) or less in thickness, the pressure of the air within the fibres is essentially atmospheric pressure and the pressure of the water on the exterior of the fibres is 137,3 mbar (—2 psig) negative pressure.

In an improved embodiment of the invention, the nest of fibres has characteristic gas pressure drop of 5 centimeters of water (5 mbar) across the length of the fibres when gas flows through the nest of fibres at the rate between 180 and 450 litres per minute.

A humidifier constructed for the average adult could be improved in having an actual characteristic flow rate of 50 litres per minute or higher, and could be further improved in that the fibres define an aggregate water-vapor transmitting surface of 0.046 m² (one half square foot).

Drawings

Fig. 1 is a longitudinal cross-sectional view of the preferred embodiment;

Fig. 2 is a transverse cross-sectional view of the embodiment taken on line 2—2 (omitting the fibers) and showing a closed mushroom valve in cross-section, while

Fig. 2a is a view of the mushroom valve when open;

Fig. 3 is a partial transverse cross-sectional view taken on line 3—3 of Fig. 1, on a magnified scale;

Fig. 4 is a longitudinal cross-sectional view on magnified scale taken on line 4—4 of Fig. 3;

Fig. 5 is a perspective view showing the embodiment in use with a patient; and

Fig. 6 is a diagrammatic view of the flow arrangement for air to be humidified and the negative pressure water.

Description of preferred embodiment

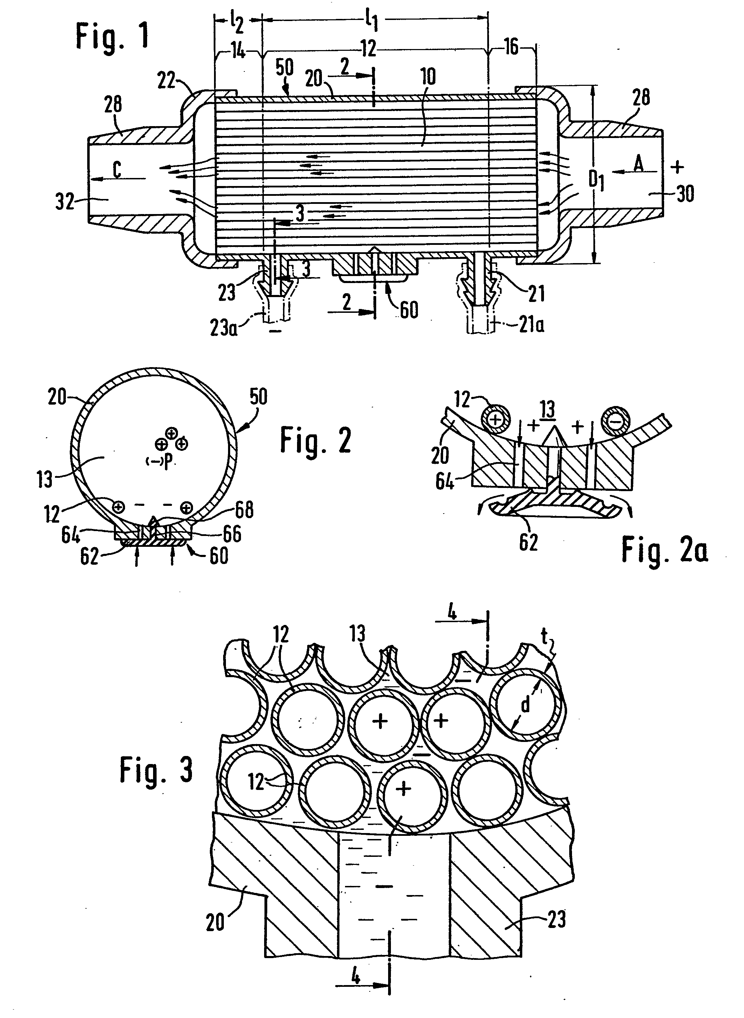

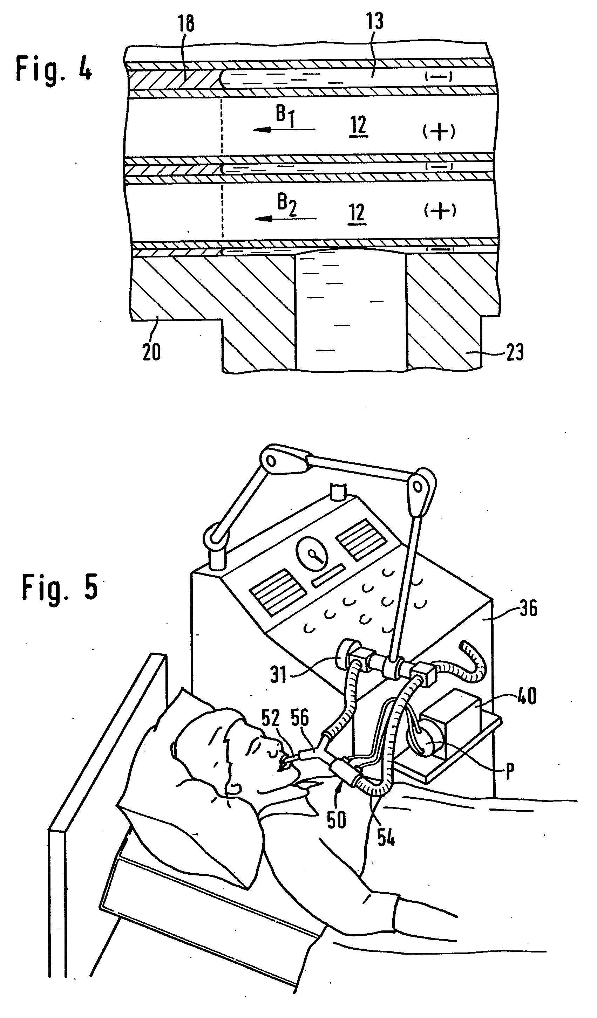

Referring to Figs. 1—4 the medical humidifier 50 comprises a bundle 10 of straight, elongated fibers, made of a substance (glycerinized polysulfone) which is permeable to water vapor and impermeable to liquid water under operating conditions. The fiber bundle is loosely arranged in its major mid-portion 12 to provide water flow passages 13 (Figs. 3 and 4) over the exterior of the fibers, while the end portions 14 and 16 of the bundle are potted (bonded together) in water-impermeable bonding material 18 (epoxy). Similarly the sides of the potted end portions 14, 16, are joined to end portions of chamber 20 defined by a rigid cylindrical wall (high density polystyrene). Between these ends the housing defines a water chamber capable of withstanding negative pressure. End caps 22 and 24 are provided at the respective ends of the housing, terminating in tapered external members 28. These serve as standard male breathing circuit connectors for insertion into mating female connectors or hose.

The humidifier unit is connected in any suitable respiratory flow path. In Fig. 5 the humidifier is shown in the inhalation leg from the respirator to a patient, with a respirator controlled exhalation valve 31 at the end of a Y connector exhausting to ambient.

As shown in Fig. 1, the end caps 22 and 24 each provide open spaces 30 and 32 beyond the ends of the fiber bundle, to define inlet and outlet air plenums. Air from the respirator 36 in Fig. 5 enters the male connector 28 at Fig. 1 at the right hand side, fills inlet air plenum 30 and is there distributed across the end face of the fiber bundle, where it splits, to enter the numerous hollow fibers as filaments of air flow. These proceed through the fibers under the pressure of the respirator, exiting into outlet air plenum 32 on the left hand side of Fig. 1 where they rejoin. The unified flow proceeds through the connector to the patient.

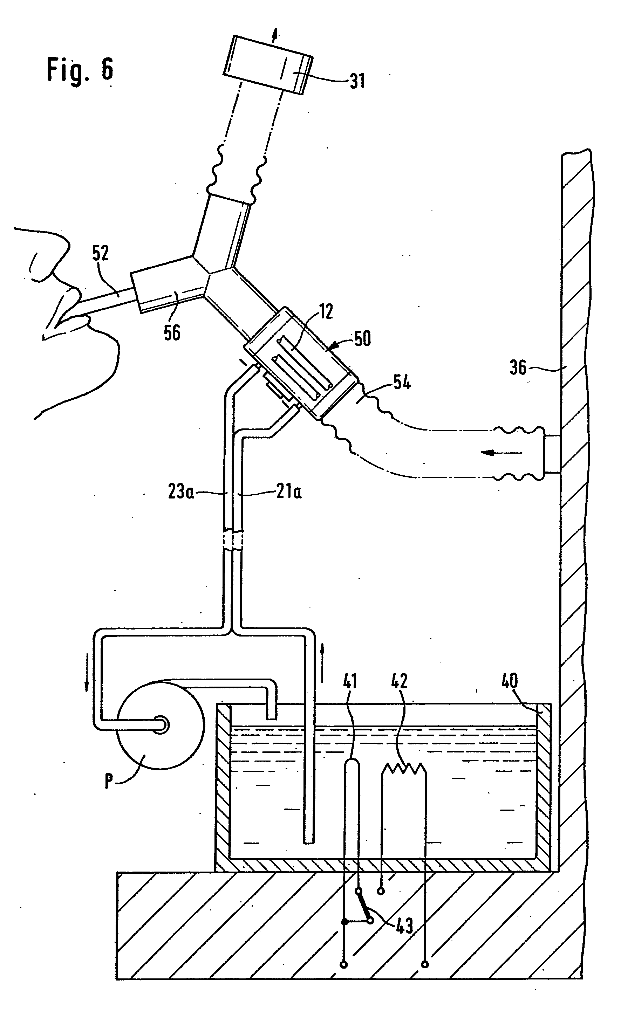

The water chamber 20 has water inlet and outlet connectors 21, 23 (of different configuration to avoid mix-up) at the right and left hand sides of Fig. 1 respectively. These connectors and the conduits, 21a, 23a are also of sufficiently rigid material to withstand negative pressure. Inlet conduit 21a extends to a water reservoir 40 (Fig. 6) where the end is submerged in water. A heater element 42 maintains the water at the desired temperature under the control of probe 41 and thermostatic control switch 43. The water outlet conduit 23a is connected to the inlet of discharge pump P which discharges excess water into the reservoir 40. Water is drawn by the pump through the inlet 21a and through the space between the loosely nested fibers, thus filling the entire free volume of chamber 20 with water under negative pressure, e.g. 137.3 m bar (—2 psig). The water is drawn through discharge conduit 23a under the pull of discharge pump P.

A positive pressure relief valve 60 (Fig. 2) is incorporated in the wall of the housing. As shown in Figs. 2 and 2a, this valve is of the so-called, well-known mushroom type.

It comprises a leaflet 62 overlying relief holes 64 which extend from the interior of the chamber 20 to the atmosphere. A stem 66 is integrally joined to leaflet 62 and ends in enlarged inward end 68 which is larger than the passage through which the stem 62 extends and therefore holds the leaflet in place. Under negative water pressure conditions as shown in Fig. 2 the leaflet seals the passages 64. However referring to the magnified view of Fig.

2a, in the event that positive pressure should accidently be applied to the water volume of chamber 20, this positive pressure acts through the passage 64 to deflect the leaflet 62 to allow escape of liquid until the positive pressure is relieved.

The fibers have internal diameter of 1.52 mm (.060 inch) and wall thickness 0.127 mm (.005 in.). They are glycerinized to make them hydrophyllic by being immersed in glycerine during spinning of the fibers, in a stage while the polysulfone is still soft.

The fibers are 7.62 cm (3 inches) in length with 0.95 to 1.27 cm (3/8 to 1/2 inch) of each end sealed in the potting material, resulting in an effective vapor-transmitting length between the ends in the range of 5.08 and 5.72 cm (2 and 2-1/4 inch).

In this embodiment 178 of the fibers are nested together and provide an aggregate vapor transmitting area in the range of 432 to 542 cm$^2$ (67 to 84 in$^2$), depending upon the length of the potted end regions, and a capacity to humidify ambient air to saturation at 37°C (98.6°F) at air flow rates between about 85 and 95 liters/min.

The flow resistance of this nested fiber module is represented by the figure of merit value

$$R = \frac{L}{D^4} \times \frac{1}{N} = 79$$

where L is the air transmitting (overall) length of the fibers in cms., D is the internal diameter of the fibers in cm and N is the number of fibers. Using this module an air flow of 300 liters/min. is achievable with an air pressure drop of 5 cm H$_2$O from end to end of the hollow fibers.

The humidifier can be placed in any of the variety of respirator circuits and flow paths and indeed the patient can spontaneously inhale through it with no assistance of a respirator.

Suitable air flow characteristics in a practical, compact module are preferably achieved with fibers ranging in internal diameter upwards from 1.27 mm (.050 inch) to about 1.78 mm (.070 inch), the length of the fibers being dependent upon their diameter and upon the number N of the fibers to be employed. The number N is also dependent upon the specific vapor transmitting characteristic of the fiber material and wall thickness selected.

The glycerinized fibers have the advantage of high vapor rate transmission and long dry shelf life and when used with the positive pressure relief valve, offer a fail-safe operation. Where even further safety is desired it is possible to use fibers which are more immune to transmission of liquid water even in the event of accidental application of positive pressure to the water chamber. In this case preferably the fibers are produced to the hardened stage as hydrophobic polysulfone dioctyl fibers and are subsequently treated with a wetting agent such as sodium sulfo succinate (stool softener marketed by Mead Johnson) and/or with glycerine. In this case, because of the added protection offered by the fibres it is in principle possible to omit the positive water pressure relief valve, but the valve is still preferred to maximize the safety of the device, particularly in view of the relatively larger number of fibers which are used when using material of the somewhat less vapor transmitting capability. The same is true if the fibers are formed of acrylic co-polymers such as the XM formulation manufactured by Amicon Corporation.

The water chamber 20 can advantageously be formed by injection molding of any suitable rigid plastic used in medical appliances, for instance the high impact polystyrene, mentioned above. The end caps, including the connectors, and defining the air plenums, can be of the same or similar material and can for instance be solvent-bonded to the exterior of the housing 20 in the manner shown. In a typical construction the assembly of fibers, having a length longer than the housing, is inserted loosely in the housing, and the potting material, epoxy or others such as silicone rubber is introduced in the end regions, through capillary action along the fibers, sometimes assisted by centrifugal force. After the potting material is set, the extreme ends of the matrix of potting material and hollow fibers can be sliced, as with a knife, flush with the ends of the housing 20, to provide a smooth end face with the fiber ends open to transmit air. The resulting bundle of fibers can readily provide a diffusion wall surface area of the order of 0.046 m$^2$ (.5 square foot), effective to humidify an aggregate air flow volume of the order of 50 to 75 liters per minute at the instant of peak flow during the respirator cycle.

Operation

Referring to the figures the gross stream of inlet air A, Fig. 1, from the respirator 36 (Fig. 6) is divided into air stream filaments B$_1$, B$_2$, etc in Figs. 3 and 4 by the many fibers of the nest. The heated water, as indicated diagrammatically in Figs. 3 and 4 flows over the exterior of these fibers while water vapor produced by this water supply permeates the thin walls of the fibers, in opposition to the pressure differential, and humidifies the dry air filaments. The air stream filaments B$_1$, B$_2$, etc. after transmitting the length of the fibers 12 are humidified to saturation at body temperature. The air filaments are then restored to a unified air flow C in the discharge plenum 32, which proceeds into the patient.

In a typical operation the respirator 36 of Fig. 5 operates as an open cycle system in which exhaled air is discharged to the atmosphere through exhalation valve 31. During the inspiration phase of the cycle the respirator gradually increases the pressure on conduit 54, supplying air through the humidifier 50 to the end of endotracheal tube 52 inserted into the airway of the patient. The valve 31 in the discharge leg 56 of the Y fitting is closed during this phase by a control line from the respirator, hence all air flow from the respirator is channeled through the humidifier 50 and into the patient.

During the expiration phase the patient exhales spontaneously. At this time valve 31 is released by the control line from the respirator to relieve the exhaled air to the atmosphere. A check valve, not shown, during the expiration phase prevents back-flow of exhaled air through the humidifier.

The hollow fibers being subjected to a decreasing pressure gradient from inside to outside take advantage of the substantial tensile strength of the wall of the fibers to ensure that they do not collapse. With the water under vacuum, little liquid water will enter the airway in the event of accidental rupture of a fiber wall. Even if a reverse pressure differential is encountered as by accidental misconnection of the pump, it is important to realize that the hollow fibers, due to their small size, demonstrate a sufficient degree of structural rigidity to resist crushing that would block the air flow to the patient.

By use of the particular fibers described, a sufficient vapor-transmitting surface area to volume ratio is obtained while still obtaining sufficient air-transmitting capacity to enable a small number of fibers, in the preferred range of 100 to 200, to be employed. The feature permits a large diffusion surface to be obtained in a small geometric volume. Such small size permits the humidifier to be mounted close to the patient, and permits it to be an inexpensive disposable component, to be replaced periodically, for instance once a day, at the same time that the hoses are ordinarily changed. The construction leads to the possibility of extending the time between sterilizations of the inlet hose, from the respirator to the humidifier, owing to the fact that it now does not contain moist warm air and therefore is not a place where bacteria multiply rapidly.

Certain features of the invention are useful without other features of the invention. For instance a humidifier using static water at atmospheric pressure as by use of a collapsible outer wall can in certain instances be used to good effect with the specified fiber construction.

## Claims

1. A humidifier for a respiratory flow path, especially medical humidifier, comprising a water filled chamber (20) including a bundle (10) of discrete elongated air transmitting hollow fibres (12) forming a compact nest and connected in parallel by inlet and outlet connections (28) and having walls of a substance permeable to water vapor, whereby the water-filled chamber (20) surrounds the effective length of the fibres, characterized in that the fibres (12) incorporate a wetting agent, the fibres (12) have a bore greater than 1,27 mm and means (P) are provided to expose the exterior of the fibres (12) to water under relatively negative pressure.

2. The humidifier of claim 1 characterized that said hollow fibres (12) have a wall thickness, that is 10% or less of the bore of said fibres.

3. The humidifier of claim 1 characterized that said hollow fibres (12) have a bore less than 1,78 mm.

4. The humidifier of claim 3, wherein the substance of said fibres (12) has a transmission characteristic of 4,8 kg water per $m^2$ per hour of fibres wall area per 0,127 mm wall thickness, with water at 37,5°C, at 137,3 mbar negative pressure on the exterior of said fibres and with a flow of anhydrous oxygen at an aggregate rate of 200 litres per minute flowing in parallel through a bundle of fibres of 127 mm length.

5. The humidifier of claim 1 characterized that the substance of said fibres (12) is selected from the group consisting of polysulfones and acrylic copolymers.

6. The humidifier of claim 1 and 5 characterized that said wetting agent is glycerine.

7. The humidifier of claim 1 characterized that said wetting agent is dioctyl sodium sulfo succinate.

8. The humidifier of claim 1, characterized that said bundle of fibres has an aggregate air flow figure of merit value R between 52 and 134 where

$$R = \frac{L}{D^4} \times \frac{1}{N}$$

L and D are length and internal diameter of fibres (12) in cm, and N is the number of fibres (12).

9. The humidifier of claim 1 or 8 characterized that the number of the fibres (12) is in the range of 100 to 200.

10. The humidifier of any of the foregoing claims characterized in that the effective length of the fibres (12) is 12,7 cm.

11. The humidifier of claim 1 characterized that said water filled chamber (20) has a positive pressure relief valve (60) which opens from the interior of the water filled chamber (20) to the atmosphere.

12. The humidifier of any of the foregoing claims characterized in that the walls of said fibres are impermeable to liquid water under operating conditions in which said walls are 0,127 mm or less in thickness, the pressure of the air within the fibres is essentially atmospheric pressure and the pressure of the water on the exterior of the fibres is 137,3 mbar negative pressure.

13. The humidifier of any of the foregoing claims characterized that the nest of fibres has a characteristic gas pressure drop of 5 centimeters of water (5 mbar) across the length of the fibres when gas flows through the nest of fibres at the rate between 180 and 450 litres per minute.

14. The humidifier of claim 13 constructed for the average adult, characterized in having an actual characteristic flow rate of 50 litres per minute or higher.

15. The humidifier of any of the foregoing claims constructed for the average adult, characterized in that the fibres define an aggregate water-vapor transmitting surface of 0.045 m².

## Revendications

1. Humidificateur destiné à être monté sur le parcours d'un gaz respiratoire, notamment humidificateur médical, comportant une chambre (20) remplie d'eau et contenant un paquet de fibres creuses distinctes (12) de forme allongée, qui conduisent l'air, forment un faisceau compact, sont reliées en parallèle par des raccords d'entrée et de sortie (28) et ont leur paroi faite d'une matière perméable à la vapeur d'eau, la chambre remplie d'eau (20) entourant les fibres sur leur longueur effective, caractérisé en ce qu'un agent mouillant est incorporé aux fibres (12), en ce que celles-ci ont un diamètre intérieur supérieur à 1,27 mm et en ce qu'il est prévu des moyens (P) pour exposer l'extérieur des fibres (12) à une dépression relative.

2. Humidificateur selon la revendication 1, caractérisé en ce que les fibres creuses (12) ont une épaisseur de paroi égale ou inférieure à 10% de leur diamètre intérieur.

3. Humidificateur selon la revendication 1, caractérisé en ce que les fibres creuses (12) ont un diamètre intérieur inférieur à 1,78 mm.

4. Humidificateur selon la revendication 3, caractérisé en ce que la matière des fibres (12) possède une caractéristique de transmission de 4,8 kg d'eau par heure et par m² de la surface de la paroi des fibres, pour une épaisseur de paroi de 0,127 mm, avec de l'eau à 37,5°C, sous une dépression de 137,3 mbar à l'extérieur des fibres et avec un courant d'oxygène anhydre circulant en parallèle à travers un paquet de fibres de 127 mm de longueur avec un débit total de 200 litres par minute.

5. Humidificateur selon la revendication 1, caractérisé en ce que la matière des fibres (12) est choisie dans un groupe comprenant les polysulfones et les copolymères acryliques.

6. Humidificateur selon les revendications 1 et 5, caractérisé en ce que l'agent mouillant est la glycérine.

7. Humidificateur selon la revendication 1, caractérisé en ce que l'agent mouillant est le sulfosuccinate de dioctyl-sodium.

8. Humidificateur selon la revendication 1, caractérisé en ce que le paquet de fibres présente un valeur globale R de résistance à l'écoulement de l'air comprise entre 52 et 134, calculée suivant la formule

$$R = \frac{L}{D^4} \times \frac{1}{N},$$

dans laquelle L et D sont respectivement la longueur et le diamètre intérieur des fibres (12) en cm et N est le nombre de fibres.

9. Humidificateur selon la revendication 1 ou 8, caractérisé en ce que le nombre de fibres (12) est compris entre 100 et 200.

10. Humidificateur selon l'une quelconque des revendications précédentes, caractérisé en ce que la longueur effective des fibres (12) est égale à 12,7 cm.

11. Humidificateur selon la revendication 1, caractérisé en ce que la chambre remplie d'eau (20) comporte une soupape de sûreté à surpression (60), qui ouvre de l'intérieur de la dite chambre (20) vers l'atmosphère.

12. Humidificateur selon l'une quelconque des revendications précédentes, caractérisé en ce que les parois des fibres sont imperméables à l'eau à l'état liquide dans des conditions de fonctionnement dans lesquelles les dites parois ont une épaisseur égale ou inférieure à 0,127 mm, la pression de l'air à l'intérieur des fibres est essentiellement égale à la pression atmosphérique et la pression de l'eau sur l'extérieur des fibres correspond à une dépression de 137,3 mbars.

13. Humidificateur selon l'une quelconque des revendications précédentes, caractérisé en ce que le faisceau de fibres a une perte de charge caractéristique de 5 cm CE (5 mbars) d'un bout à l'autre de la longueur des fibres lorsqu'il est traversé par le gaz à raison de 180 à 450 litres par minute.

14. Humidificateur selon la revendication 13, conçu pour l'adulte moyen, caractérisé en ce qu'il a un débit caractéristique réel égal ou supérieur à 50 litres par minute.

15. Humidificateur selon l'une quelconque des revendications précédentes, conçu pour l'adulte moyen, caractérisé en ce que les fibres définissent une surface globale de transmission de la vapeur d'eau égale à 0,045 m².

## Patentansprüche

1. Anfeuchter für einen Atemluftströmungsweg, insbesondere, medizinischer Anfeuchter, mit einer wassergefüllten Kammer (20) welche ein Bündel (10) von getrennten in Längsrichtung erstreckten, luftführenden Hohlfasern (12) enthält, die ein kompaktes Bündel bilden und durch Einlaß- und Auslaßverbindungen (28) parallel geschaltet sind und deren Wandteile aus einem Material bestehen, welches für Wasserdampf durchlässig ist, und bei dem die wassergefüllte Kammer (20) die wirksame Länge der Fasern umgibt, dadurch gekennzeichnet, daß die Fasern (12) eine benetzende Substanz enthalten, daß die Fasern (12) eine Bohrung größer als 1,27 mm aufweisen und daß Mittel (P) vorgesehen sind, um das Äußere der Fasern (12) unter erheblichem Unterdruck stehendem Wasser auszusetzen.

2. Anfeuchter nach Anspruch 1, dadurch gekennzeichnet, daß die Hohlfasern (12) eine Wandstärke aufweisen, die 10% oder weniger der Bohrung der Fasern entspricht.

3. Anfeuchter nach Anspruch 1, dadurch gekennzeichnet, daß die Hohlfasern (12) eine Bohrung von weniger als 1,78 mm aufweisen.

4. Anfeuchter nach Anspruch 3, dadurch gekennzeichnet, daß das Material der Fasern (12) eine Durchlässigkeitscharakteristik von 4,8 kg Wasser pro m$^2$ und Stunde im Faserwandbereich bei 0,127 mm Wandstärke aufweist, mit Wasser von 37,5°C bei 137,3 mbar Unterdruck an der Außenseite der Fasern und bei einer Strömung von wasserfreiem Sauerstoff mit einem Gesamtdurchfluß von 200 l pro Minute in paralleler Durchströmung eines Hohlfaserbündels von 127 mm Länge.

5. Anfeuchter nach Anspruch 1, dadurch gekennzeichnet, daß das Material der Fasern (12) aus der Gruppe der Polysulfone und der Acrylcopolymere ausgewählt ist.

6. Anfeuchter nach Anspruch 1 und 5, dadurch gekennzeichnet, daß die benetzende Substanz Glycerin ist.

7. Anfeuchter nach Anspruch 1, dadurch gekennzeichnet, daß die benetzende Substanz Dioctylnatriumsulfosuccinat ist.

8. Anfeuchter nach Anspruch 1, dadurch gekennzeichnet, daß das Faserbündel einen Gesamtdurchflußin solcher Größenordnung aufweist, daß der Wert von R zwischen 52 und 134 liegt, wobei

$$R = \frac{L}{D^4} \times \frac{1}{N}$$

bedeutet, und wobei mit L und D Länge und innerer Durchmesser der Fasern (12) in cm, und mit N die Anzahl der Fasern (12) bezeichnet werden.

9. Anfeuchter nach Anspruch 1 oder 8, dadurch gekennzeichnet, daß die Anzahl der Fasern (12) im Bereich von 100 bis 200 liegt.

10. Anfeuchter nach einem der vorangehenden Ansprüche, dadurch gekennzeichnet, daß die wirksame Länge der Fasern (12) 12,7 cm beträgt.

11. Anfeuchter nach Anspruch 1, dadurch gekennzeichnet, daß die wassergefüllte Kammer (20) ein auf positiven Druck ansprechendes Sicherheitsventil (60) aufweist, welches vom Innenraum der wassergefüllten Kammer (20) nach der Atmosphäre öffnet.

12. Anfeuchter nach einem der vorangehenden Ansprüche dadurch gekennzeichnet, daß die Wandungen der Fasern für flüssiges Wasser unter Betriebsbedingungen undurchlässig sind, daß die Wandstärke 0,127 mm oder weniger beträgt, daß der Druck der Luft innerhalb der Fasern im wesentlichen der atmosphärische Luftdruck ist, und daß der Druck des Wassers auf der äusseren Oberfläche der Fasern ein Unterdruck von 137,3 mbar ist.

13. Anfeuchter nach einem der vorangehenden Ansprüche, dadurch gekennzeichnet, daß das Faserbündel einen charakteristischen Gasdruckabfall von 5 cm Wassersäule längs der Fasern aufweist, wenn das Gas mit einem Durchfluß zwischen 180 und 450 l pro Minute durch das Faserbündel strömt.

14. Anfeuchter nach Anspruch 13, in der Ausführung für den durchschnittlichen Erwachsenen, dadurch gekennzeichnet, daß der tatsächliche charakteristische Durchfluß bei 50 l pro Minute oder darüber liegt.

15. Anfeuchter nach einem der vorangehenden Ansprüche in der Ausführung für den durchschnittlichen Erwachsenen, dadurch gekennzeichnet, daß die Fasern eine gesamte wasserdampfdurchlässige Oberfläche von 0.045 m$^2$ bilden.

0 009 543

**Fig. 1**

**Fig. 2**

**Fig. 2a**

**Fig. 3**

Fig. 4

Fig. 5

Fig. 6